# EUROPÄISCHE PATENTSCHRIFT

(11) **EP 3 185 215 B1**
(45) Veröffentlichungstag und Bekanntmachung des Hinweises auf die Patenterteilung: **15.08.2018**
(21) Anmeldenummer: 16199100.5
(22) Anmeldetag: 16.11.2016
(51) Int. Cl.: G06T 11/00, G06T 7/12, G06T 7/41

(54) **AUTOMATISIERTE ERMITTLUNG VON KONTUREN AUF BASIS EINER ITERATIVEN REKONSTRUKTION**
AUTOMATED DETERMINATION OF CONTOURS ON THE BASIS OF ITERATIVE RECONSTRUCTION
DÉTERMINATION AUTOMATISÉE DE CONTOURS SUR LA BASE D'UNE RECONSTRUCTION ITÉRATIVE

(30) Priorität: 22.12.2015 DE 102015226400
(43) Veröffentlichungstag der Anmeldung: 28.06.2017
(73) Patentinhaber: Siemens Healthcare GmbH, 91052 Erlangen (DE)
(72) Erfinder: Raupach, Rainer, 91336 Heroldsbach (DE); Soza, Grzegorz, 90562 Heroldsberg (DE)

(56) Entgegenhaltungen:
- US-A1- 2014 161 352
- YOON SUNGWON ET AL: "Simultaneous segmentation and reconstruction: A level set method approach for limited view computed tomography", MEDICAL PHYSICS, AIP, MELVILLE, NY, US, Bd. 37, Nr. 5, 29. April 2010 (2010-04-29) , Seiten 2329-2340, XP012135763, ISSN: 0094-2405, DOI: 10.1118/1.3397463
- HARALICK ET AL.: "Textural Features for Image Classification", IEEE TRANSACTIONS ON SYSTEMS, MAN AND CYBERNETICS, Bd. SMC-3, Nr. 6, November 1973 (1973-11), Seiten 610-621, XP011192771,
- Jalda Dworzak: "3D reconstruction of the human rib cage from 2D projection images using a statistical shape model", Diplomarbeit, 1 December 2009 (2009-12-01), pages x-i-73, XP055454660, Berlin, Germany Retrieved from the Internet: URL:https://www.zib.de/lamecker/research/D worzak-Diplom.pdf [retrieved on 2018-02-27]

## Beschreibung

Die Erfindung betrifft ein Verfahren zum Rekonstruieren von Bilddaten und Texturmerkmalen eines abzubildenden Untersuchungsbereichs. Weiterhin betrifft die Erfindung ein Verfahren zum Segmentieren eines abzubildenden Untersuchungsbereichs. Überdies betrifft die Erfindung eine Rekonstruktionseinrichtung. Außerdem betrifft die Erfindung eine Bildsegmentierungseinrichtung. Zudem betrifft die Erfindung ein Computertomographiesystem.

Die Segmentierung von Organen in vorgegebenen Bildern von medizinischen bildgebenden Geräten ist ein entscheidender Schritt bei vielen klinischen Anwendungen. Beispielsweise ist die Segmentierung der Leber ein notwendiger Schritt, um deren Volumen oder Teilvolumen davon zu ermitteln. Die Kenntnis dieser Informationen kann dann genutzt werden, um zum Beispiel exakte Operationsschnitte während einer Leberoperation zu planen.

Ein anderes Beispiel betrifft das Ermitteln von Konturen von Organen mit einer hohen Strahlungsempfindlichkeit, um eine Bestrahlungstherapiebehandlung zu planen. In diesem Zusammenhang ist es wichtig, empfindliche gesunde anatomische Strukturen, wie zum Beispiel die Leber oder die Blase, im Körper eines Patienten zu kennzeichnen, um diese vor einer Schädigung durch die bei der therapeutischen Bestrahlung auftretende Strahlenexposition zu bewahren. Die segmentierten gesunden Strukturen und die segmentierten zu bestrahlenden Tumore werden dann mit in die Konzeption eines Bestrahlungstherapieplans einbezogen, so dass für den Patienten ein optimales Ergebnis hinsichtlich des Gesundheitsrisikos und des Nutzens der Bestrahlung erzielt wird.

Jalda Dworzak, "3D Reconstruction of the Human Rib Cage from 2D Projection Images Using a Statistical Shape Model", Diplomarbeit, Zuse-Institut Berlin, 2009, XP055454660, offenbart ein Verfahren zur dreidimensionalen Rekonstruktion der Lage und Form des menschlichen Brustkorbes aus wenigen zwei-dimensionalen Projektionsbildern. Die Rekonstruktionsmethode basiert auf einem statistischen 3D-Formmodell der Rippen, das zum Zwecke der Rekonstruktion in eine Bildebene projiziert und an die 2D-Bilddaten eines individuellen Brustkorbes angepasst wird. Diese Anpassung wird durch die Minimierung eines Distanzmaßes gesteuert, das Unterschiede zwischen Projektionen des Modells und den 2D-Projektionsdaten misst.

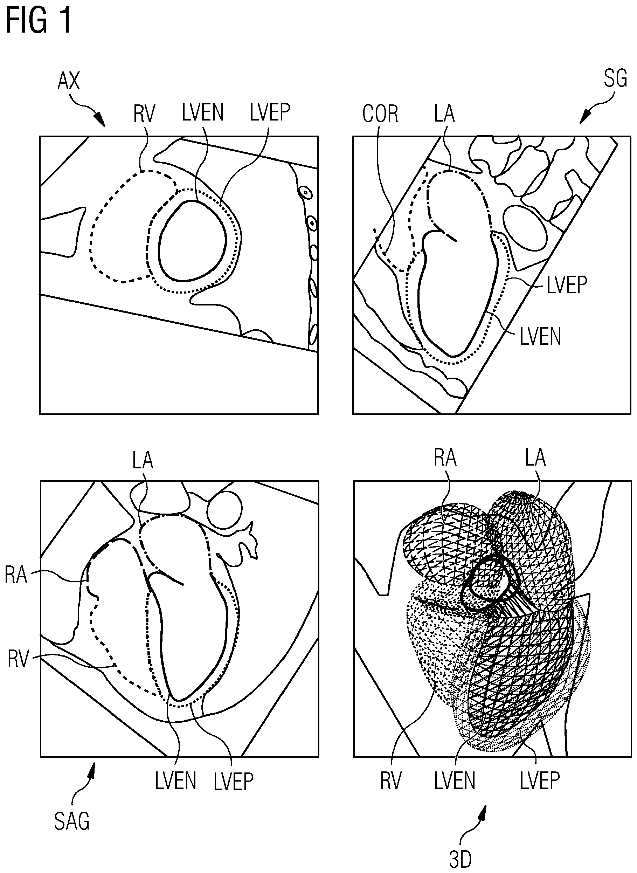

Es ist auf jeden Fall wünschenswert, ein automatisiertes Segmentierungsverfahren zu entwickeln, welches ein schnelles und exaktes Bearbeiten von umfangreichen Datenmengen erlaubt. Bisher wurden einige Anwendungen mit modernen Bildverarbeitungsmethoden automatisiert. Beispielsweise ist eine automatisierte Leber- oder Herz-Segmentierung Bestandteil vieler klinischer Anwendungen. Allerdings sind die bereits vorhandenen Lösungen nicht perfekt und erfordern - um ein korrektes Ergebnis zu erzielen - bei einem Teil der Segmentierungsvorgänge immer noch ein Eingreifen des Benutzers. Es gibt auch eine Vielzahl von Anwendungsfällen, bei denen der Segmentierungsvorgang vollständig von Hand vorgenommen wird, indem anatomische Objekte mit einfachen geometrischen Werkzeugen im Zweidimensionalen konturiert werden und dann zu dreidimensionalen Strukturen kombiniert werden (siehe z.B. FIG 1).

Ein Problem besteht darin, dass die Grenzen zwischen unterschiedlichen anatomischen Objekten nicht immer klar zu erkennen sind und auch aktuelle Algorithmen nicht in der Lage sind, solche Objekte perfekt zu segmentieren. Beispielsweise haben bei nicht kontrastierten CT-Bilddaten Voxel, welche zur Leber gehören, dieselben CT-Werte (Hounsfield-Werte) wie die Voxel, welche benachbartem Muskelgewebe zugeordnet sind.

Um eine Segmentierung auch auf der Basis von Bilddaten durchführen zu können, bei denen im Bilddatenraum Grenzen zwischen verschiedenen anatomischen Strukturen nicht klar erkennbar sind, wurden herkömmlich vorab bekannte Informationen über die Bilddaten in die angewandten Segmentierungs-Algorithmen mit einbezogen. Eine besonders neuartige Vorgehensweise ist dabei das sogenannte maschinelle Lernen, wobei ein statistisches Computermodell erzeugt wird, welches geometrische Merkmale und Texturmerkmale umfasst und auf Basis einer großen Anzahl von Bilddaten ermittelt wird. Ein solches Modell wird dann auf eine Patientenanatomie angewandt, wobei individuell Patienteninformationen in den aufgenommenen Bilddaten berücksichtigt werden. Eine solche Vorgehensweise ermöglicht es, mit Bildabschnitten besser umzugehen, bei denen eine visuelle Differenzierung nicht möglich ist. In diesen Bildabschnitten wird die Modellgeometrie verwendet, um einen Mangel an Bildkontrast zu kompensieren. Allerdings wurden auch mit solchen statistischen Modellen nicht alle Segmentierungsprobleme gelöst. Der Grund dafür ist darin zu finden, dass in den Bilddaten fehlende Information nicht exakt durch statistische Informationen kompensiert werden kann.

Während der Bildaufnahme mit Hilfe von medizinischen Bildgebungseinrichtungen werden in einem ersten Schritt Rohdaten, auch Mess-Projektionsdaten genannt, erfasst, welche zum Beispiel bei Computertomographie-Geräten der Absorption von Röntgenstrahlen in Abhängigkeit von unterschiedlichen Projektionswinkeln entsprechen. Anschließend werden auf Basis der Rohdaten unter Anwendung von Integrationsverfahren Bilddaten rekonstruiert. Bei allen herkömmlichen Segmentierungsverfahren beschränken sich alle Modellansätze auf die Voxelinformation der Bilddaten aus dem Bilddatenraum. Jedoch geht bei der Transformation von den Rohdaten zu den rekonstruierten Bilddaten ein erheblicher Anteil von Informationen verloren und kann allein anhand der Bilddaten auch nicht rekonstruiert werden.

Es besteht somit das Problem, ein universell einsetzbares, automatisiertes Rekonstruktionsverfahren anzugeben, welches eine verbesserte Genauigkeit und Zuverlässigkeit bei der Ermittlung von anatomischen Strukturen, insbesondere von Konturen von anatomischen Strukturen, aufweist.

Diese Aufgabe wird durch ein Verfahren zum Rekonstruieren von Bilddaten und Texturmerkmalen eines abzubildenden Untersuchungsbereichs gemäß Patentanspruch 1, ein Verfahren zum Segmentieren eines abzubildenden Untersuchungsbereichs gemäß Patentanspruch 4, eine Bildrekonstruktionseinrichtung gemäß Patentanspruch 11, eine Bildsegmentierungseinrichtung gemäß Patentanspruch 12 und ein Computertomographiesystem gemäß Patentanspruch 13 gelöst.

Bei dem erfindungsgemäßen Verfahren zum Rekonstruieren von Bilddaten und Texturmerkmalen eines abzubildenden Untersuchungsbereichs werden Mess-Projektionsdaten von dem abzubildenden Untersuchungsbereich erfasst. Wie bereits erwähnt, kann es sich bei der Anwendung des Verfahrens im Zusammenhang mit der Bildgebung unter Verwendung von Computertomographie-geräten bei den Mess-Projektionsdaten um Messwerte der Absorption von Röntgenstrahlen in Abhängigkeit von unterschiedlichen Projektionswinkeln handeln. Anschließend wird auf Basis der erfassten Mess-Projektionsdaten eine iterative Rekonstruktion durchgeführt.

Die iterative Rekonstruktion ist ein Rekonstruktionsverfahren zur Rekonstruktion von Bilddaten aus erfassten Mess-Projektionsdaten, insbesondere bei der Computertomographie. Mit dieser Rekonstruktionsmethode können Bilddaten mit geringem Rauschanteil erzeugt werden. Bei diesem Verfahren wird für den Bilddatenrekonstruktionsprozess eine Korrekturschleife angewendet, in der Schnittbilder eins Computertomogramms schrittweise durch eine allmähliche Annäherung an die tatsächliche Dichteverteilung bzw. Verteilung von Schwächungswerten in einem Aufnahmebereich errechnet werden. Dazu wird zunächst eine Annahme über die Dichteverteilung der in dem Aufnahmebereich vorhandenen zu untersuchenden Gewebeschichten getroffen und ein Ausgangsbild berechnet. Aus diesem Ausgangsbild werden neue, synthetische Projektionsdaten erzeugt und mit den tatsächlich aufgenommenen "echten" Mess-Projektionsdaten, auch Rohdaten genannt, verglichen. Stimmen sie nicht überein, wird ein entsprechendes Korrekturbild berechnet, mit dessen Hilfe das Ausgangsbild korrigiert wird. Dann werden erneut Projektionsdaten synthetisiert und mit den gemessenen Rohdaten verglichen. Diese Iteration wird so lange fortgesetzt, bis ein definiertes Abbruchkriterium erfüllt ist. Danach ist im korrigierten Bild die räumliche Bildauflösung in kontrastreichen Regionen erhöht, das Bildrauschen in gering kontrastierten Arealen ist dagegen reduziert. Das Bild wird in dichte-homogenen Geweberegionen weicher, während kontrastreiche Gewebegrenzen erhalten bleiben. Bildauflösung und Bildrauschen sind entkoppelt.

Bei der iterativen Rekonstruktion werden nun erfindungsgemäß vorläufige Bilddaten und vorläufige Texturmerkmale gewonnen. Diese Texturmerkmale entsprechen individuellen Charakteristiken einzelner Punkte im Projektionsdatenraum und werden bei der iterativen Rekonstruktion teilweise mitberechnet. Die Texturmerkmale werden an den Grenzen zwischen zwei anatomischen Objekten, aber auch in den anderen Bereichen innerhalb der anatomischen Objekte berechnet. Diese Texturinformationen sind bei der herkömmlichen iterativen Rekonstruktion allerdings nicht mehr in den bei der iterativen Rekonstruktion resultierenden endgültigen Bilddaten vorhanden. Jedoch umfassen diese Texturdaten wertvolle Informationen, wie zum Beispiel die Wahrscheinlichkeit, dass ein bestimmter Punkt im Raum Teil einer Grenzlinie zwischen zwei anatomischen Objekten ist. Erfindungsgemäß werden nun diese Texturmerkmale für die Gewinnung von zusätzlichen Strukturinformationen, vorzugsweise Konturinformationen, genutzt.

Die Strukturinformationen umfassen vorzugsweise Konturinformationen, also Informationen bezüglich des Verlaufs von Grenzen zwischen anatomischen Strukturen. Sie können aber auch Informationen hinsichtlich der Strukturen innerhalb von Organen bzw. allgemein anatomischen Objekten oder Bereichen umfassen. Diese Strukturen können zum Beispiel Tumorstrukturen oder ähnliches sein, die innerhalb eines Organs erkannt werden sollen.

Im Rahmen des erfindungsgemäßen Verfahrens können auch zusätzliche Texturmerkmale, die nicht für das Erzeugen der regulären Bilddaten benötigt werden, auf Basis der Rohdaten berechnet werden, um die Qualität von zu gewinnenden Strukturinformationen, vorzugsweise Konturen zu verbessern. Solche zusätzlichen Texturmerkmale können zum Beispiel Kanten oder Grenzlinien zwischen anatomischen Objekten betreffen, sie können aber auch in Beziehung mit einer charakteristischen Textur innerhalb eines Objekts stehen. Texturmerkmale geben Auskunft über die Verteilung von Graustufen in einer Bildaufnahme. Eine ausführliche Beschreibung dieser Texturmerkmale ist in Haralick et al. "Textural Features for Image Classification", IEEE Transactions on Systems, Man and Cybernetics, Vol. SMC-3, No. 6, November 1973, pp.610-621, zu finden, welche hiermit durch Bezug in die vorliegende Patentanmeldung einbezogen ist.

Auf Basis der gewonnenen vorläufigen Texturmerkmale werden nun erwartete vorläufige Strukturen, vorzugsweise Konturen, in dem Untersuchungsbereich ermittelt. Beispielsweise umfassen die Texturmerkmale Informationen, welche mit einer Wahrscheinlichkeit korreliert sind, dass eine Kante oder Kontur an einer bestimmten Stelle im Bild vorhanden ist. Auf Basis dieser Informationen werden dann die vorläufigen Strukturen, vorzugsweise Konturen, im Bilddatenraum festgelegt. Anschließend werden die erwarteten vorläufigen Strukturen, vorzugsweise Konturen, bei dem nächsten Iterationsschritt der iterativen Rekonstruktion mitberücksichtigt. D. h., sie fließen mit ein in die Erzeugung von synthetischen Projektionsdaten und werden anschließend bei dem Vergleich zwischen den synthetischen Projektionsdaten und den Rohdaten sowie bei der Erzeugung von Korrekturbilddaten und analog auch von korrigierten Texturmerkmalen mitberücksichtigt. Insbesondere werden die erwarteten vorläufigen Strukturen, beispielsweise Konturen, im Rahmen der iterativen Rekonstruktion bei einer Filterung im Rohdatenraum berücksichtigt. Die erwarteten vorläufigen Strukturen stellen separate Informationen dar, die erfindungsgemäß bei der Filterung berücksichtigt werden. Auf diese Weise werden die Strukturinformationen schrittweise und damit verbunden die Texturmerkmale präzisiert. Werden die ermittelten Konturinformationen mit den rekonstruierten Bilddaten kombiniert, so erhält man ein Bild mit den jeweiligen anatomischen Objekten zugeordneten, klar abgegrenzten Bildbereichen.

Vorteilhaft werden bei dem erfindungsgemäßen Verfahren die bei der Rekonstruktion miterzeugten Texturmerkmale in den iterativen Prozess der Bildrekonstruktion und der Rekonstruktion von Texturmerkmalen automatisiert mit einfließen gelassen. Die Texturmerkmale umfassen wertvolle Informationen hinsichtlich des Auftretens von Strukturen, insbesondere Konturen, wie zum Beispiel Grenzlinien zwischen Organen. Im Gegensatz zu herkömmlichen Verfahren werden diese Informationen bei der Bildrekonstruktion zusätzlich mit erzeugt und gehen nach der iterativen Rekonstruktion mit ein in eine anschließende Analyse und Weiterverarbeitung der Bilddaten, insbesondere eine anschließende Segmentierung der Bilddaten. Auf diese Weise wird ein präziseres Rekonstruktionsverfahren erreicht, welches eine prägnante Darstellung auch von Bildbereichen ermöglicht, die im Bilddatenraum keine starken Kontraste aufweisen.

Bei dem erfindungsgemäßen Verfahren zum Segmentieren eines abzubildenden Untersuchungsbereichs wird zunächst das erfindungsgemäße Verfahren zum Rekonstruieren von Bilddaten und Texturmerkmalen eines abzubildenden Untersuchungsbereichs durchgeführt. Anschließend wird der abzubildende Untersuchungsbereich unter Berücksichtigung von auf Basis der vorläufigen Texturmerkmale ermittelten Texturmerkmalen segmentiert. Das erfindungsgemäße Verfahren ist insbesondere auch auf Bildbereiche anwendbar, die sich allein mit Hilfe von statistischen Modellen nicht gut segmentieren lassen. Die beschriebenen Schritte laufen vorzugsweise automatisiert ab. Ein Eingreifen eines Benutzers, wie zum Beispiel ein Segmentieren durch Einzeichnen von Konturen, ist bei dem erfindungsgemäßen Verfahren nicht nötig.

Die bei der iterativen Rekonstruktion am Ende resultierenden Texturmerkmale weisen ähnlich wie die iterativ rekonstruierten Bilddaten eine hohe Zuverlässigkeit und Präzision auf und können zum Beispiel sehr exakte Angaben über die Wahrscheinlichkeit des Auftretens von Grenzlinien zwischen anatomischen Objekten enthalten. Diese Informationen werden bei der automatisierten Segmentierung zur Festlegung von Grenzlinien zwischen einzelnen anatomischen Objekten verwendet. Alternativ zu einer Segmentierung kann auch eine Extraktion von in Bilddaten mit Hilfe der Texturmerkmale ermittelten Strukturen bzw. Strukturbereiche erfolgen. Dabei werden mit Hilfe der Texturmerkmale Bildbereich mit bestimmten Strukturen ermittelt, welche zur genaueren Analyse aus den Bilddaten extrahiert werden.

Die erfindungsgemäße Bildrekonstruktionseinrichtung umfasst eine Projektionsdatenerfassungseinheit zum Erfassen von Mess-Projektionsdaten. Teil der erfindungsgemäßen Bildrekonstruktionseinrichtung ist außerdem eine Bilddatenrekonstruktionseinheit zum Durchführen einer iterativen Rekonstruktion von vorläufigen Bilddaten auf Basis der erfassten Mess-Projektionsdaten. Bei der iterativen Rekonstruktion werden zusätzlich zu den vorläufigen Bilddaten vorläufige Texturmerkmale gewonnen. Weiterhin umfasst die erfindungsgemäße Bildrekonstruktionseinrichtung auch eine Strukturermittlungseinheit zum Ermitteln von erwarteten vorläufigen Strukturen in einem abzubildenden Untersuchungsbereich eines Untersuchungsobjekts auf Basis der gewonnenen vorläufigen Texturmerkmale.

Die erfindungsgemäße Bildsegmentierungseinrichtung weist die erfindungsgemäße Rekonstruktionseinrichtung auf. Außerdem weist die erfindungsgemäße Bildsegmentierungseinrichtung auch eine Segmentierungseinheit auf, welche dazu eingerichtet ist, die rekonstruierten Bilddaten unter Berücksichtigung von auf Basis der vorläufigen Texturmerkmale ermittelten Texturmerkmalen zu segmentieren. Mit Hilfe der erfindungsgemäßen Bildsegmentierungseinrichtung können erfasste anatomische Bilder automatisiert segmentiert werden, wobei Grenzlinien zwischen verschiedenen anatomischen Bereichen, beispielsweise Organen, in den anatomischen Bildern auf Basis der automatisiert ermittelten Strukturen, vorzugsweise Konturen, automatisiert eingezeichnet werden.

Das erfindungsgemäße Computertomographiesystem weist eine erfindungsgemäße Bildrekonstruktionseinrichtung auf. Insbesondere in der Computertomographie werden aus verschiedenen Winkeln erzeugte Projektionsdaten zu Bilddaten rekonstruiert. Bei dieser Art der medizinischen Bildgebung findet die erfindungsgemäße Bildrekonstruktionseinrichtung besonders bevorzugt Anwendung, da sie zur Bildrekonstruktion das bei der Computertomographie häufig zum Einsatz kommende iterative Rekonstruktionsverfahren nutzt.

Die wesentlichen Komponenten der erfindungsgemäßen Bildrekonstruktionseinrichtung können zum überwiegenden Teil in Form von Softwarekomponenten ausgebildet sein. Dies betrifft insbesondere Teile der Bildrekonstruktionseinrichtung, wie zum Beispiel die Bilddatenrekonstruktionseinheit und die Strukturermittlungseinheit. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, wie zum Beispiel die Projektionsdatenerfassungseinheit - beispielsweise, wenn es nur um eine Übernahme von Daten aus anderen Softwarekomponenten geht - als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete Bildrekonstruktionseinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer erfindungsgemäßen Bildrekonstruktionseinrichtung ladbar ist, mit Programmabschnitten, um alle Schritte des erfindungsgemäßen Verfahrens auszuführen, wenn das Computerprogramm in der Bildsegmentierungseinrichtung ausgeführt wird.

Ein solches Computerprogrammprodukt kann neben dem Computerprogramm gegebenenfalls zusätzliche Bestandteile wie z.B. eine Dokumentation und/oder zusätzliche Komponenten, auch Hardware-Komponenten, wie z.B. Hardware-Schlüssel (Dongles etc.) zur Nutzung der Software, umfassen

Zum Transport zur Speichereinrichtung der Bildrekonstruktionseinrichtung und/oder zur Speicherung an der Bildrekonstruktionseinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Bildrekonstruktionseinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung auch die verschiedenen Merkmale unterschiedlicher Ausführungsbeispiele und Ansprüche auch zu neuen Ausführungsbeispielen kombiniert werden.

In einer Ausgestaltung des erfindungsgemäßen Verfahrens zum Rekonstruieren von Bilddaten und Texturmerkmalen eines abzubildenden Untersuchungsbereichs erfolgt das Ermitteln der erwarteten vorläufigen Strukturen in dem Untersuchungsbereich zusätzlich auf Basis der rekonstruierten vorläufigen Bilddaten. Bei dieser Ausgestaltung können also zum Beispiel sowohl Bilddaten als auch Texturmerkmale in einer vorbestimmten Gewichtung in die Ermittlung von vorläufigen Konturen eingehen.

Um eine verlässliche Grundlage für eine Beziehung zwischen den Texturmerkmalen und vorherzusagenden Strukturen, vorzugsweise Konturen, zu erhalten, kann zum Beispiel auch ein Strukturen-Vorhersagemodell verwendet werden. Ein solches Strukturen-Vorhersagemodell erlaubt es, auf Basis allgemeiner Modellannahmen die ermittelten Texturmerkmale in Beziehung mit zu erwartenden Konturen zu setzen.

In einer besonders vorteilhaft anzuwendenden Variante des erfindungsgemäßen Verfahrens zum Rekonstruieren von Bilddaten und Texturmerkmalen eines abzubildenden Untersuchungsbereichs werden die Schritte der iterativen Rekonstruktion so lange wiederholt, bis ein Qualitätskriterium für die ermittelten vorläufigen Bilddaten und vorläufigen Texturmerkmale erfüllt ist. Somit wird eine Mindestqualität der ermittelten Bild- und Strukturdaten garantiert.

Unter wiederholter Anwendung der Rekonstruktionsschritte nähern sich die rekonstruierten Bilddaten und Texturmerkmale einer gewünschten Präzision allmählich an. Das Qualitätskriterium kann zum Beispiel die Erkennbarkeit von auf Basis der ermittelten Bilddaten und Texturmerkmale zu ermittelnden Strukturen, vorzugsweise Konturen, das Signal/Rausch-Verhältnis und die Auflösung in einzelnen Bildbereichen der Bilddaten umfassen.

In einer bevorzugten Ausgestaltung des erfindungsgemäßen Verfahrens zum Segmentieren eines abzubildenden Untersuchungsbereichs erfolgt das Segmentieren des abzubildenden Untersuchungsbereichs zusätzlich unter Berücksichtigung von auf der Basis der rekonstruierten vorläufigen Bilddaten ermittelten Bilddaten. Bei dieser Variante werden also bei der Segmentierung zusätzlich zu den ermittelten Texturmerkmalen auch mit einer vorbestimmten Gewichtung berücksichtigte Bilddaten berücksichtigt.

Bei dem Schritt der Segmentierung kann außerdem ein Konturen-Vorhersagemodell angewendet werden, um auf Basis der ermittelten Texturmerkmale und der ermittelten Bilddaten endgültige, möglichst verlässliche Konturen festzulegen, welche dann zur Segmentierung der Bilddaten bzw. der auf Basis der Bilddaten erzeugten anatomischen Bilder genutzt werden. Ein Konturen-Vorhersagemodell kann zum Beispiel selbstorganisiert und basierend auf einer Datenbasis erzeugt werden und eine Relation zwischen den bei der Konturenbildung berücksichtigten Daten und den Konturen selbst umfassen.

In einer besonders vorteilhaften Ausgestaltung des erfindungsgemäßen Verfahrens zum Rekonstruieren von Bilddaten und Texturmerkmalen eines abzubildenden Untersuchungsbereichs oder des erfindungsgemäßen Verfahrens zum Segmentieren eines abzubildenden Untersuchungsbereichs wird ein Strukturen-Vorhersagemodell mit Hilfe eines Trainingsverfahrens gewonnen. Bei dem Trainingsverfahren werden zunächst Trainings-Mess-Projektionsdaten erfasst. Die Trainings-Mess-Projektionsdaten können zum Beispiel von einer Datenbank stammen oder direkt durch ein bildgebendes Verfahren von einem Untersuchungsobjekt erfasst werden. Anschließend wird auf Basis der Trainings-Mess-Projektionsdaten eine iterative Rekonstruktion von vorläufigen Trainings-Bilddaten durchgeführt. Dabei werden bei der iterativen Rekonstruktion zusätzlich vorläufige Trainings-Texturmerkmale ermittelt. Zwischen einzelnen Rekonstruktionsschritten werden nun zusätzlich Strukturen, vorzugsweise Konturen, in den vorläufigen Trainings-Bilddaten annotiert. Das heißt, es werden zum Beispiel Grenzlinien zwischen anatomischen Strukturen, deren Position dem Einzeichnenden bekannt ist, in den Bilddaten eingezeichnet.

Danach erfolgt ein erneutes iteratives Rekonstruieren von vorläufigen Trainings-Bilddaten und Ermitteln von vorläufigen Trainings-Texturmerkmalen unter Berücksichtigung der annotierten Strukturen, insbesondere der annotierten Konturen. Das heißt, die annotierten Strukturen werden bei der Ermittlung von synthetisierten Projektionsdaten im Rahmen des iterativen Rekonstruktionsschritts ebenfalls mitberücksichtigt und fließen somit auch in die Rekonstruktion von neuen vorläufigen Trainings-Bilddaten und vorläufigen Trainings-Texturmerkmalen ein. Nach mehrmaliger Iteration werden die zuletzt rekonstruierten vorläufigen Trainings-Bilddaten und die zuletzt gewonnenen vorläufigen Trainings-Texturmerkmale als endgültige Trainings-Bilddaten und endgültige Trainings-Texturmerkmale eingestuft. Die endgültigen Trainings-Bilddaten und die endgültigen Trainings-Texturmerkmale werden schließlich dazu verwendet, um ein Strukturen-Vorhersagemodell zu erzeugen.

Ein solches Strukturen-Vorhersagemodell, vorzugsweise Konturen-Vorhersagemodell, gibt eine Beziehung zwischen rekonstruierten Bilddaten, Texturmerkmalen und Strukturen, insbesondere Konturen, an. Mit Hilfe des Strukturen-Vorhersagemodells kann also auf Basis der Texturmerkmale beispielsweise auf die Position von Objektgrenzen oder bestimmter Strukturen geschlossen werden. Diese Informationen können bei dem erfindungsgemäßen Bildrekonstruktionsverfahren und dem erfindungsgemäßen Segmentierungsverfahren wiederum dazu genutzt werden, lokale Texturmerkmale genauer zu ermitteln. Beispielsweise kann im Rahmen der iterativen Rekonstruktion bei dem erfindungsgemäßen Bildrekonstruktionsverfahren ein anisotropes Filtern längs der angenommenen Objektgrenzen derart ausgeführt werden, dass die Kanten kontrastiert und scharf abgebildet werden. Da bei dem Trainingsverfahren die genannte Beziehung zwischen Strukturen, insbesondere Konturen, und Texturmerkmalen noch nicht bekannt ist, werden die Strukturen, vorzugsweise Konturen, in den Trainings-Bilddaten per Hand eingezeichnet. Anschließend werden die Strukturen mit den aktuellen vorläufigen Trainings-Bilddaten in den Projektionsdatenraum transformiert und bei dem nächsten Iterationsschritt bzw. dem nächsten Rekonstruktionsschritt mitberücksichtigt. Bei dem nächsten Rekonstruktionsschritt werden dann wieder neue vorläufige Trainings-Bilddaten sowie neue vorläufige Trainings-Texturmerkmale rekonstruiert, die, was die Darstellung von Strukturen, insbesondere Konturen, betrifft, bereits verbessert sind. Auf diese Weise werden präzise Trainings-Texturmerkmale sowie präzise Trainings-Bilddaten erzeugt, die anschließend zur Bildung eines Strukturen-Vorhersagemodells genutzt werden können.

Im Rahmen einer bevorzugten Variante des erfindungsgemäßen Verfahrens zum Rekonstruieren von Bilddaten und Texturmerkmalen eines abzubildenden Untersuchungsbereichs oder des erfindungsgemäßen Verfahrens zum Segmentieren eines abzubildenden Untersuchungsbereichs werden die Schritte des Annotierens von Strukturen, des erneuten iterativen Rekonstruierens von vorläufigen Trainings-Bilddaten und des Ermittelns von vorläufigen Trainings-Texturmerkmalen so oft wiederholt, bis ein Qualitätskriterium der ermittelten vorläufigen Trainings-Bilddaten und der ermittelten vorläufigen Trainings-Texturmerkmale erfüllt ist. Das Qualitätskriterium kann zum Beispiel einen Kontrast oder eine Auflösung der Bilddaten sowie eine Verlässlichkeit der aufgenommenen Texturmerkmale beinhalten. Auf diese Weise kann eine geforderte Mindestqualität der Bilddarstellung sowie der Konturen der erfassten Bilddaten erreicht werden.

In einer besonders praktikablen Variante des erfindungsgemäßen Verfahrens zum Rekonstruieren von Bilddaten und Texturmerkmalen eines abzubildenden Untersuchungsbereichs oder des erfindungsgemäßen Verfahrens zum Segmentieren eines abzubildenden Untersuchungsbereichs wird zur Erzeugung des Strukturen-Vorhersagemodells auf Basis der endgültigen Trainings-Bilddaten und der endgültigen Trainings-Texturmerkmale ein maschineller Lernprozess durchgeführt. Ein solcher maschineller Lernprozess ist zum Beispiel in Bengio et al., 2007, "Layer-Wise Training of Deep Networks", Advances in Neural Information Processing Systems, beschrieben. Alternativ zu dem maschinellen Lernprozess kann auch ein als "deep learning approach" (siehe Zheng et al, 2007, "Fast Automatic Heart Chamber Segmentation from 3D CT Data using Marginal Space Learning and Steerable Features", In Proc. Intl. Conf. Computer Vision) bezeichneter Lernprozess zum Einsatz kommen. Vorteilhaft wird das Strukturen-Vorhersagemodell auf Basis einer Statistik, d. h. einer Vielzahl von vorliegenden Trainingsdaten, selbstorganisiert ermittelt. D. h., es wird keine starre Modellstruktur für die Beziehung zwischen einzelnen Größen vorgegeben, sondern die Modellstruktur entsteht erst auf Basis der verwendeten Trainingsdaten und erlaubt somit eine flexible Anpassung an den jeweiligen Einsatzbereich.

In einer speziellen Variante des erfindungsgemäßen Verfahrens zum Rekonstruieren von Bilddaten und Texturmerkmalen eines abzubildenden Untersuchungsbereichs oder des erfindungsgemäßen Verfahrens zum Segmentieren eines abzubildenden Untersuchungsbereichs umfassen die Texturmerkmale "edge maps", die im Deutschen als Kantenbilder bezeichnet werden und im Bilddatenraum vorliegen. Diese Kantenbilder kombinieren für jeden Voxel eines oder mehrere Texturmerkmale, welche bei dem iterativen Rekonstruktionsprozess ermittelt wurden. Sie unterscheiden sich von herkömmlichen Bilddaten und können eher als Konturbilder betrachtet werden. Beispielsweise kann ein Kantenbild nur die reinen Konturen eines Bildes umfassen. Bei einer komplexeren Ausgestaltung kann ein solches Kantenbild auch die Konturen kombiniert mit den rekonstruierten Bilddaten umfassen.

Ein Kantenbild wird aus einem digitalen Bild durch sogenannte Kantendetektion berechnet, indem benachbarte Bildelemente auf Änderungen ihrer Farb- oder Grauwerte analysiert werden. Als Ergebnis werden markante Farb- und Helligkeitsübergänge weiß markiert. Alle übrigen Bildbereiche sind schwarz. Die in den Kantenbildern enthaltenen Konturinformationen können bei dem erfindungsgemäßen Verfahren zur Ermittlung von Konturen in den bei der iterativen Rekonstruktion erzeugten Bilddaten verwendet werden.

In einer Ausgestaltung des erfindungsgemäßen Verfahrens zum Rekonstruieren von Bilddaten und Texturmerkmalen eines abzubildenden Untersuchungsbereichs oder des erfindungsgemäßen Verfahrens zum Segmentieren eines abzubildenden Untersuchungsbereichs weisen die Texturmerkmale Texturmerkmal-Vektoren auf. Die sogenannten Texturmerkmal-Vektoren sind multidimensionale Vektoren, welche jeweils einen Punkt im Rekonstruktionsraum, d.h. dem Bilddatenraum, betreffen und deren Komponenten Texturmerkmale umfassen.

Sowohl die Kantenbilder als auch die Texturmerkmal-Vektoren können im Rahmen des Trainingsverfahrens von einem der genannten Lernalgorithmen zur Erzeugung eines Strukturen-Vorhersagemodells, insbesondere eines Konturen-Vorhersagemodells, genutzt werden. In einem solchen Lernprozess werden von Hand erzeugte Referenz-Segmentierungen, welche von erfahrenen Radiologen vorgenommen wurden, auf die Trainings-Bilddaten angewandt, um den Zusammenhang zwischen Texturmerkmalen und Konturen von Organgrenzen und inneren und äußeren Bereichen zu lernen. D. h., es wird eine Relation zwischen den Trainingsbilddaten zugeordneten Texturmerkmalen und den in dem Trainingsprozess annotierten Konturen ermittelt.

In einer Ausgestaltung der erfindungsgemäßen Bildsegmentierungseinrichtung ist die Strukturen-Ermittlungseinheit, vorzugsweise eine Konturen-Ermittlungseinheit dazu eingerichtet, bei dem Ermitteln von erwarteten vorläufigen Konturen in einem abzubildenden Untersuchungsbereich eines Untersuchungsobjekts zusätzlich zu den vorläufigen Texturmerkmalen ein vorzugsweise trainiertes Konturen-Vorhersagemodell anzuwenden. Wie bereits erwähnt, erlaubt ein solches Konturen-Vorhersagemodell eine zuverlässige Ermittlung von Konturen auf Basis der rekonstruierten Bilddaten und Texturmerkmale.

Die erfindungsgemäße Bildsegmentierungseinrichtung weist bevorzugt eine Konturen-Vorhersagemodell-Trainingseinheit zum Erzeugen eines trainierten Konturen-Vorhersagemodells auf. Eine solche Konturen-Vorhersagemodell-Trainingseinheit ermöglicht das Trainieren eines Konturen-Vorhersagemodells mit der erfindungsgemäßen Bildsegmentierungseinrichtung, so dass keine zusätzlichen Einrichtungen für den beschriebenen Trainingsprozess benötigt werden. Wie bereits erwähnt, kann die erfindungsgemäße Bildsegmentierungseinrichtung zumindest teilweise auch als Software-Lösung implementiert sein. Die erfindungsgemäße Bildsegmentierungseinrichtung kann nun leicht mit Hilfe von zusätzlichen Softwareprogrammen auch dazu befähigt werden, die beschriebenen Trainingsprozesse zu unterstützen.

Die Erfindung wird im Folgenden unter Hinweis auf die beigefügten Figuren anhand von Ausführungsbeispielen noch einmal näher erläutert. Es zeigen:
- FIG 1: eine beispielhafte Darstellung einer Segmentierung eines menschlichen Herzens,
- FIG 2: ein Blockdiagramm, welches ein Verfahren zum Ermitteln von Konturdaten gemäß einem Ausführungsbeispiel der Erfindung zeigt,
- FIG 3: ein Blockdiagramm, welches die Erzeugung eines Konturen-Vorhersagemodells mit Hilfe eines Trainingsverfahrens veranschaulicht,
- FIG 4: eine schematische Darstellung einer Bildsegmentierungseinrichtung gemäß einem Ausführungsbeispiel der Erfindung,
- FIG 5: eine schematische Darstellung eines Computertomographiesystems gemäß einem Ausführungsbeispiel der Erfindung.

In FIG 1 ist eine Segmentierung eines menschlichen Herzens zur Veranschaulichung der bei der Segmentierung üblichen Vorgehensweise in vier Teilbildern dargestellt. Die Darstellung umfasst drei zweidimensionale Bildaufnahmen bzw. Schnittbilder, in denen Teilorgangrenzen zwischen Teilorganen des Herzens eingezeichnet sind. Ein viertes Teilbild umfasst eine dreidimensionale segmentierte Darstellung des Herzens. Ein erstes Teilbild ist in einer axialen Ebene AX aufgenommen. In dem ersten Teilbild sind zweidimensionale Konturen einer rechten Herzkammer RV (auch rechtes Ventrikel genannt), einer Innenwand LVEN einer linken Herzkammer und einer Außenwand LVEP der linken Herzkammer gezeigt. Ein zweites Teilbild ist in einer cordialen Ebene COR aufgenommen. In dem zweiten Teilbild sind zusätzlich zu den Umrissen der rechten Herzkammer RV, der Innenwand LVEN der linken Herzkammer und der Außenwand LVEP der linken Herzkammer Umrisse des linken Herzvorhofs LA eingezeichnet. Ein drittes Teilbild ist in einer sagittalen Ebene SAG aufgenommen. In dem dritten Teilbild sind zusätzlich zu den Umrissen der rechten Herzkammer RV, der Innenwand LVEN der linken Herzkammer und der Außenwand LVEP der linken Herzkammer und den Umrissen des linken Herzvorhofs LA auch Umrisse des rechten Herzvorhofs RA eingezeichnet. In dem vierten Teilbild 3D sind die Umrisse bzw. Begrenzungen aller in den zweidimensionalen Bildern bereits mit Umrissen gekennzeichneten Teilorgane, also insbesondere die Umrisse der rechten Herzkammer RV, der Innenwand LVEN der linken Herzkammer, der Außenwand LVEP der linken Herzkammer, des linken Herzvorhofs LA und des rechten Herzvorhofs RA, als Flächennetze im dreidimensionalen Raum dargestellt. Mit Hilfe des vierten Teilbilds können nun Therapieplanungen, wie zum Beispiel die Planung einer Herzoperation, vorgenommen werden, wobei die Grenzen einzelner Teilorgane anhand des vierten Teilbilds bekannt sind und so zum Beispiel die Position und Länge von Skalpellschnitten recht genau vorherbestimmt werden kann.

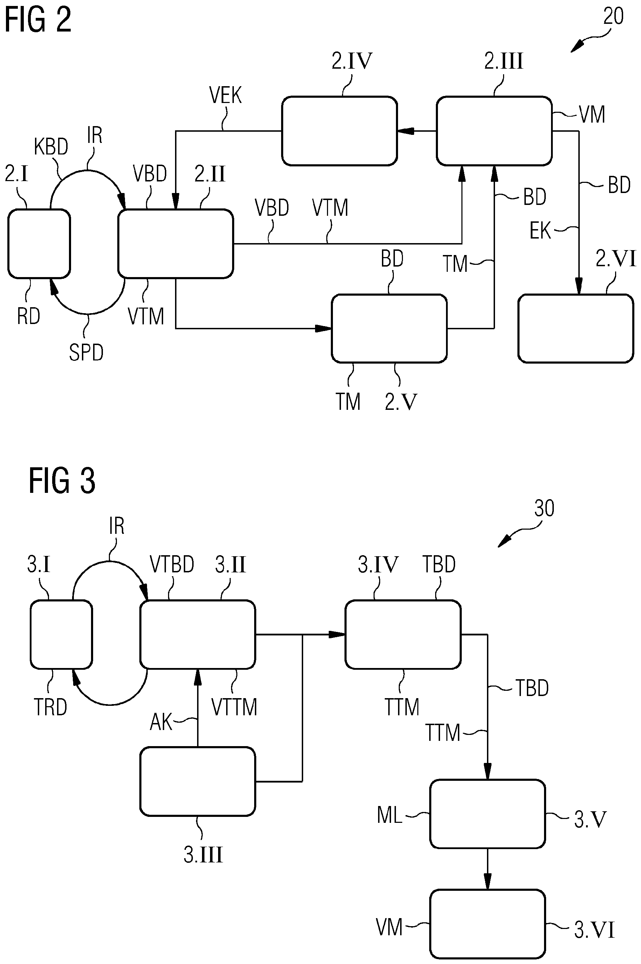

In FIG 2 ist eine schematische Darstellung 20 gezeigt, welche ein Verfahren zum Segmentieren eines abzubildenden Untersuchungsbereichs gemäß einem Ausführungsbeispiel der Erfindung veranschaulicht. Bei dem in FIG 2 gezeigten Verfahren erfolgt eine Segmentierung eines individuellen Bilddatensatzes BD unter Anwendung eines Vorhersagemodells VM, welches zum Beispiel gemäß dem in FIG 3 veranschaulichten Verfahren erzeugt wurde.

Bei dem Schritt 2.I werden zunächst Rohdaten RD, auch Mess-Projektionsdaten genannt, von einem abzubildenden Untersuchungsbereich FOV erfasst. Dies kann zum Beispiel mit Hilfe einer computertomographischen Bildaufnahme geschehen. Die erfassten Rohdaten werden nun bei dem Schritt 2.II einem ersten Rekonstruktionsschritt unterzogen, welcher Bestandteil eines iterativen Rekonstruktionsvorgangs IR ist. Bei dem ersten Rekonstruktionsschritt werden zusätzlich zu vorläufigen Bilddaten VBD auch vorläufige Texturmerkmale VTM, beispielsweise in Form von multidimensionalen Texturmerkmalvektoren, ermittelt. Anschließend werden bei dem Schritt 2.III auf Basis eines Vorhersagemodells VM und der vorläufigen Texturmerkmale VTM Wahrscheinlichkeiten bzw. Wahrscheinlichkeitswerte für in den vorläufigen Bilddaten VBD vorhandene Konturen ermittelt. Das heißt, die Wahrscheinlichkeitswerte geben Auskunft darüber, welche Konturen an welcher Stelle in Abhängigkeit von den vorläufigen Texturmerkmalen VTM und den vorläufigen Bilddaten zu erwarten sind. Bei dem Schritt 2.IV werden dann auf Basis der ermittelten Wahrscheinlichkeiten erwartete vorläufige Konturen VEK in den vorläufigen Bilddaten VBD festgelegt. Anschließend wird zu dem Schritt 2.II zurückgekehrt und auf Basis der vorläufigen Bilddaten VBD, der vorläufigen Texturmerkmale VTM und der vorläufigen erwarteten Konturen VEK ein erneuter Rekonstruktionsschritt durchgeführt. Bei einem solchen Rekonstruktionsschritt werden, wie bereits anfangs erwähnt, synthetisierte Projektionsdaten SPD ermittelt, welche dann mit den erfassten Rohdaten RD verglichen werden. Auf Basis dieses Vergleichs werden anschließend Korrekturbilddaten KBD erzeugt, welche dazu dienen, die bisher rekonstruierten vorläufigen Bilddaten VBD zu korrigieren. Bei dem Rekonstruktionsschritt werden zum Beispiel bei einem Filterungsprozess auch die ermittelten vorläufigen erwarteten Konturen VEK berücksichtigt. Dabei erfolgt beispielsweise eine "Glättung" nur in Längsrichtung von vorläufigen Kanten und Grenzlinien und nicht quer dazu. Erfüllen die bei der Rekonstruktion erzeugten korrigierten vorläufigen Bilddaten VBD ein vorbestimmtes Qualitätskriterium, wie zum Beispiel eine Sichtbarkeit von Konturen, so wird zu dem Schritt 2.V übergegangen, bei dem die zuletzt ermittelten vorläufigen Bilddaten VBD und die zuletzt ermittelten vorläufigen Texturmerkmale VTM als die endgültigen Bilddaten BD und die endgültigen Texturmerkmale TM eingestuft werden. Anschließend wird zu dem Schritt 2.III zurückgekehrt, wobei endgültige Konturen EK auf Basis der endgültigen Bilddaten BD und der endgültigen Texturmerkmale TM und unter Anwendung des Vorhersagemodells VM ermittelt werden. Schließlich erfolgt bei dem Schritt 2.VI eine Segmentierung der rekonstruierten Bilddaten BD unter Anwendung der ermittelten endgültigen Konturen EK auf die endgültigen Bilddaten BD. Das heißt, es erfolgt eine Segmentierung der Bilddaten BD entlang der ermittelten Konturen EK. Damit werden insbesondere Objektgrenzen auf Bildern, welche auf Basis der Bilddaten BD erzeugt wurden, kenntlich gemacht.

In FIG 3 ist ein Blockdiagramm gezeigt, welches die Erzeugung eines Vorhersagemodells mit Hilfe eines Trainingsverfahrens veranschaulicht. In der Trainingsphase soll ein Vorhersagemodell unter Anwendung eines maschinellen Lernprozesses erzeugt werden. Bei dem Schritt 3.I werden zunächst Trainings-Mess-Projektionsdaten TRD erfasst. Die Trainings-Mess-Projektionsdaten TRD können zum Beispiel aus einer Datenbank entnommen sein oder direkt durch eine Bildaufnahme von einem Patienten erzeugt worden sein. Bei dem Schritt 3.II wird anschließend ein erster Rekonstruktionsschritt im Rahmen einer iterativen Rekonstruktion IR von vorläufigen Trainings-Bilddaten VTBD auf Basis der Trainings-Mess-Projektionsdaten TRD vorgenommen. Dabei werden zusätzlich zu den vorläufigen Trainings-Bilddaten VTBD auch vorläufige Trainings-Texturmerkmale VTTM ermittelt. In diesem Stadium sind sowohl die vorläufigen Trainings-Bilddaten VTBD als auch die vorläufigen Trainings-Texturmerkmale VTTM noch ungenau. Um Konturen in den vorläufigen Trainings-Bilddaten VTTM deutlicher zu machen, werden nun bei einem Schritt 3.III Objektgrenzen als Konturen AK, beispielsweise von Hand, in den Trainings-Bilddaten VTBD eingetragen bzw. annotiert. Um die annotierten Konturdaten AK bei dem nächsten Rekonstruktionsschritt berücksichtigen zu können, werden diese vom Bilddatenraum in den Rohdatenraum zurücktransformiert.

Anschließend wird zu dem Schritt 3.II zurückgekehrt, wobei nun jedoch der nächste Rekonstruktionsschritt unter Berücksichtigung der annotierten Konturdaten AK ausgeführt wird. Beispielsweise wird bei der Rekonstruktion ein anisotropes Filtern längs der eingezeichneten Konturen AK derart ausgeführt, dass Kanten in den Bilddaten nicht unscharf werden, sondern eher verstärkt werden. Das heißt, ein bei der iterativen Rekonstruktion angewandter Filter-Algorithmus berücksichtigt die annotierten Konturen AK. Die bei dem Schritt 3.II erneut erzeugten vorläufigen Trainings-Bilddaten VTBD und zugehörigen Trainings-Texturmerkmale VTTM sind nun bereits deutlich präziser als bei dem ersten Durchgang, da bei ihrer Rekonstruktion die annotierten Konturinformationen AK berücksichtigt wurden.

Anschließend können bei einem erneuten Schritt 3.III in den erhaltenen vorläufigen Trainings-Bilddaten VTBD wiederum Objektgrenzen als annotierte Konturdaten AK eingezeichnet werden. Diese können dann bei einem weiteren Rekonstruktionsschritt 3.II auf die bereits beschriebene Art und Weise in die Rekonstruktion von vorläufigen Trainings-Bilddaten VTBD und zugehörigen Trainings-Texturmerkmalen VTTM mit einfließen. Ist eine gewünschte Bildqualität erreicht, bei der Objektgrenzen ausreichend sichtbar sind, so erfolgt bei dem Schritt 3.IV eine Einstufung der zuletzt rekonstruierten vorläufigen Trainings-Bilddaten VTBD und der zuletzt gewonnenen vorläufigen Trainings-Texturmerkmale VTTM als endgültige Trainings-Bilddaten ETBD und endgültige Trainings-Texturmerkmale ETTM.

Nachfolgend wird bei dem Schritt 3.V ein spezieller Trainingsprozess ML durchgeführt, wobei die rekonstruierten Bilddaten TBD und Texturmerkmale TTM mit manuell von Experten in Bilddaten eingezeichneten Referenzsegmentierungen verglichen werden. Werden entsprechend viele Trainings-Bilddatensätze TBD und Referenzsegmentierungen berücksichtigt, so kann eine Statistik ermittelt werden, mit deren Hilfe zum Beispiel Organgrenzen, inneren Bereichen von Organen und Bereichen außerhalb der Organe bestimmte Texturmerkmale zugeordnet werden. Für den Trainingsprozess im Rahmen des Schritts 3.V kann zum Beispiel ein sogenannter maschineller Lernprozess zum Einsatz kommen. Die bei dem Schritt 3.V ermittelte statistische Beziehung zwischen den Texturmerkmalen und den Organgrenzen bzw. Konturen wird bei dem Schritt 3.VI dazu genutzt, ein Vorhersagemodell VM zu erzeugen, welches zu Bilddaten aus einem gewünschten Untersuchungsbereich und eventuell bekannten Texturmerkmalen eine Vorhersage bezüglich der Position der in dem Untersuchungsbereich vorhandenen Objektgrenzen bzw. Konturen vornehmen kann.

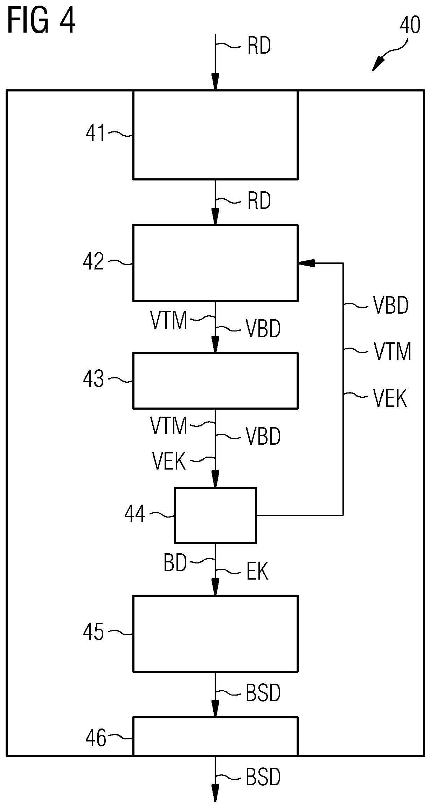

In FIG 4 ist eine Bildsegmentierungseinrichtung 40 gemäß einem Ausführungsbeispiel der Erfindung gezeigt. Die Bildsegmentierungseinrichtung 40 umfasst eine Projektionsdatenerfassungseinheit 41 zum Erfassen von Mess-Projektionsdaten RD, welche zum Beispiel im Rahmen eines CT-Bildgebungsverfahrens von einem Untersuchungsbereich FOV aufgenommen wurden. Die erfassten Rohdaten RD werden an eine Bilddatenrekonstruktionseinheit 42 weitergeleitet, welche auf Basis der erfassten Rohdaten RD im Rahmen eines ersten Schritts einer iterativen Rekonstruktion vorläufige Bilddaten VBD rekonstruiert. Bei dem Rekonstruktionsschritt werden zusätzlich zu den vorläufigen Bilddaten VBD auch vorläufige Texturmerkmale VTM gewonnen. Die vorläufigen Bilddaten VBD sowie die vorläufigen Texturmerkmale VTM werden anschließend an eine Konturermittlungseinheit 43 übermittelt, welche auf Basis der gewonnenen vorläufigen Texturmerkmale VTM erwartete vorläufige Konturen VEK in dem abzubildenden Untersuchungsbereich FOV ermittelt. Danach werden die ermittelten Daten VTM, VEK, VBD an eine Qualitätskontrolleinheit 44 übermittelt, welche auf Basis der ermittelten Daten prüft, ob die vorläufigen Bilddaten VBD bereits eine vorbestimmte Qualität erreicht haben und die vorläufigen Konturen VEK eine vorgegebene minimale Zuverlässigkeit aufweisen. Falls das noch nicht der Fall ist, erfolgt unter Berücksichtigung der ermittelten vorläufigen Konturen VEK sowie der ermittelten vorläufigen Bilddaten VBD ein weiterer iterativer Bilddatenrekonstruktionsschritt durch die Bilddatenrekonstruktionseinheit 42. Dabei werden erneut vorläufige Bilddaten VBD und vorläufige Texturmerkmale VTM erzeugt.

Anschließend werden die rekonstruierten Daten VBD, VTM von der Konturermittlungseinheit 43 wiederum zur Ermittlung von angepassten Konturdaten VEK genutzt. Sollte nachfolgend die Qualitätskontrolleinheit 44 auf Basis der neu ermittelten Daten VBD, VTM, VEK feststellen, dass die genannten Qualitätskriterien erfüllt sind, so werden die ermittelten vorläufigen Bilddaten VBD und vorläufigen Konturdaten VEK als endgültige Bilddaten BD und endgültige Konturdaten EK eingestuft und an eine Segmentierungseinheit 45 weitergeleitet. Die Segmentierungseinheit 45 verwendet die ermittelten Konturdaten EK, um die rekonstruierten Bilddaten BD zu segmentieren. Dabei werden beispielsweise Organgrenzen identifiziert und in den Bilddaten eingezeichnet. Die dabei erzeugten Bildsegmentdaten BSD können anschließend über eine Ausgangsschnittstelle 46 an eine Datenbank oder zum Beispiel an eine Bildschirmeinheit zur weiteren Bearbeitung und Analyse ausgegeben werden.

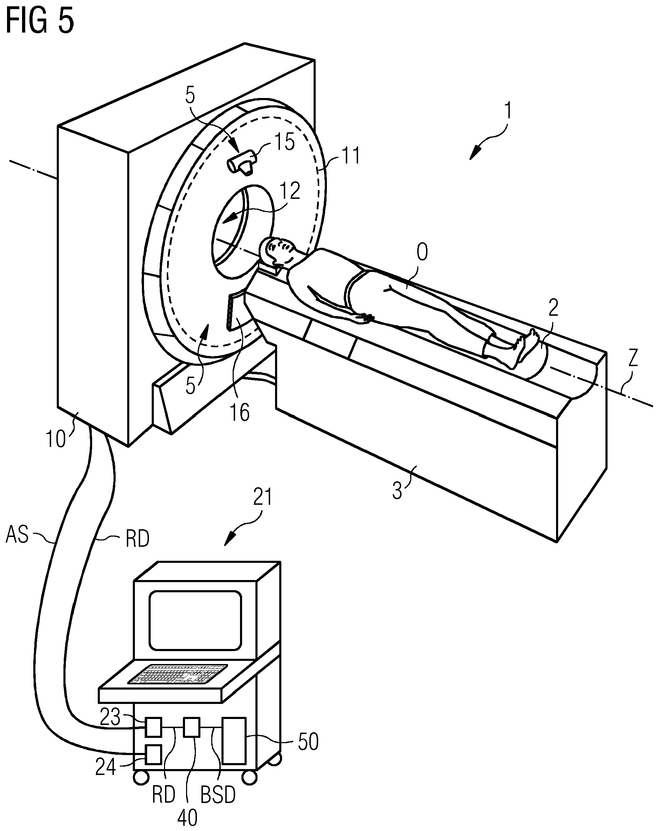

In FIG 5 ist schematisch ein Computertomographiesystem (CT-System) 1 mit einer erfindungsgemäßen Bildsegmentierungseinrichtung 40 gemäß einem Ausführungsbeispiel der Erfindung dargestellt.

Das CT-System 1 besteht dabei im Wesentlichen aus einem Scanner 10, in welchem an einer Gantry 11 eine Projektionsdatenakquisitionseinheit 5 mit einem Detektor 16 und einer dem Detektor 16 gegenüberliegenden Röntgenquelle 15 um einen Messraum 12 umläuft. Vor dem Scanner 10 befindet sich eine Patientenlagerungseinrichtung 3 bzw. ein Patiententisch 3, dessen oberer Teil 2 mit einem darauf befindlichen Patienten O zum Scanner 10 verschoben werden kann, um den Patienten O durch den Messraum 12 hindurch relativ zum Detektorsystem 16 zu bewegen. Angesteuert werden der Scanner 10 und der Patiententisch 3 durch eine Steuereinrichtung 21, von der aus über eine übliche Steuerschnittstelle 24 Akquisitionssteuersignale AS kommen, um das gesamte System gemäß vorgegebener Messprotokolle in der herkömmlichen Weise anzusteuern. Durch die Bewegung des Patienten O entlang der z-Richtung, welche der Systemachse z längs durch den Messraum 12 entspricht, und den gleichzeitigen Umlauf der Röntgenquelle 15 ergibt sich für die Röntgenquelle 15 relativ zum Patienten O während der Messung eine Helixbahn. Parallel läuft dabei immer gegenüber der Röntgenquelle 15 der Detektor 16 mit, um Projektionsmessdaten RD zu erfassen, die dann zur Rekonstruktion von Volumen- und/ oder Schicht-Bilddaten genutzt werden. Ebenso kann auch ein sequentielles Messverfahren durchgeführt werden, bei dem eine feste Position in z-Richtung angefahren wird und dann während eines Umlaufs, eines Teilumlaufs oder mehrerer Umläufe an der betreffenden z-Position die erforderlichen Projektionsmessdaten RD erfasst werden, um ein Schnittbild an dieser z-Position zu rekonstruieren oder um aus den Projektionsdaten mehrerer z-Positionen Volumenbilddaten zu rekonstruieren. Das erfindungsgemäße Verfahren ist grundsätzlich auch an anderen CT-Systemen, z.B. mit einem einen vollständigen Ring bildenden Detektor, einsetzbar.

Die vom Detektor 16 akquirierten Mess-Projektionsdaten RD (im Folgenden auch Rohdaten genannt) von einem Untersuchungsbereich FOV werden über eine Rohdatenschnittstelle 23 an die Steuereinrichtung 21 übergeben. Diese Rohdaten werden dann in einer Bildsegmentierungseinrichtung 40 weiterverarbeitet, die bei diesem Ausführungsbeispiel in der Steuereinrichtung 21 in Form von Software auf einem Prozessor realisiert ist. Diese Bildsegmentierungseinrichtung 40 führt auf Basis der erfassten Rohdaten RD eine Bildsegmentierung auf die im Zusammenhang mit FIG 2 beschriebene Art und Weise durch.

Anschließend werden die segmentierten Bilddaten BSD an eine Speichereinrichtung 50 weitergegeben. Von dort können die Bildinformationen BSD beispielsweise auf einem Bildschirm graphisch dargestellt werden oder auch an externe Analyseeinrichtungen oder Therapieplanungseinrichtungen (nicht gezeigt) weitergegeben werden.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. So wurden das Verfahren, die Bildrekonstruktionseinrichtung und die Bildsegmentierungseinrichtung in erster Linie anhand eines Systems zur Aufnahme von medizinischen Bilddaten erläutert. Die Erfindung ist jedoch nicht auf eine Anwendung im medizinischen Bereich beschränkt, sondern kann grundsätzlich auch auf die Rekonstruktion und Segmentierung von Bildern für andere Zwecke angewandt werden. Weiterhin wurde das erfindungsgemäße Verfahren im Zusammenhang mit der Rekonstruktion und Segmentierung von CT-Bildern erläutert. Die Erfindung ist jedoch nicht auf die Rekonstruktion und Segmentierung von CT-Bildern beschränkt, sondern kann auch auf andere bildgebende Verfahren angewandt werden. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Verfahren zum Rekonstruieren von Bilddaten (BD) und Texturmerkmalen (TM) eines abzubildenden Untersuchungsbereichs (FOV), welches die Schritte aufweist:
- Erfassen von Mess-Projektionsdaten (RD) von dem abzubildenden Untersuchungsbereich (FOV),
- Durchführen einer iterativen Rekonstruktion (IR) auf Basis der erfassten Mess-Projektionsdaten (RD), wobei im Rahmen der iterativen Rekonstruktion (IR) folgende Schritte erfolgen:
- Rekonstruieren von vorläufigen Bilddaten (VBD) und vorläufigen Texturmerkmalen (VTM),
- Ermitteln von erwarteten vorläufigen Strukturen, vorzugsweise erwarteten vorläufigen Konturen (VEK), in dem Untersuchungsbereich (FOV) auf Basis der gewonnenen vorläufigen Texturmerkmale (VTM),
- Berücksichtigen der erwarteten vorläufigen Strukturen bei der iterativen Rekonstruktion von vorläufigen Bilddaten (VBD) und vorläufigen Texturmerkmalen (VTM).
- Festlegen der sich aus der iterativen Rekonstruktion zuletzt ergebenden vorläufigen Bilddaten (VBD) und vorläufigen Texturmerkmale (VTM) als die rekonstruierten Bilddaten (BD) und Texturmerkmale (TM).

2. Verfahren nach Anspruch 1, wobei das Ermitteln der erwarteten vorläufigen Strukturen (VEK) in dem Untersuchungsbereich (FOV) zusätzlich auf Basis der rekonstruierten vorläufigen Bilddaten (VBD) und/oder eines Strukturen-Vorhersagemodells (VM) erfolgt.

3. Verfahren nach Anspruch 1 oder 2, wobei die Schritte im Rahmen der iterativen Rekonstruktion (IR) so lange wiederholt werden, bis ein Qualitätskriterium für die ermittelten vorläufigen Bilddaten (VBD) und vorläufigen Texturmerkmale (VTM) erfüllt ist.

4. Verfahren zum Segmentieren eines abzubildenden Untersuchungsbereichs (FOV), aufweisend die Schritte:
- Rekonstruieren von Bilddaten (BD) und Texturmerkmalen (TM) unter Anwendung des Verfahrens nach einem der Ansprüche 1 bis 3,
- Segmentieren des abzubildenden Untersuchungsbereichs (FOV) unter Berücksichtigung der ermittelten Texturmerkmale (TM).

5. Verfahren nach Anspruch 4, wobei das Segmentieren des abzubildenden Untersuchungsbereichs (FOV) zusätzlich unter Berücksichtigung der ermittelten Bilddaten (BD) und/oder eines Strukturen-Vorhersagemodells, vorzugsweise eines Konturen-Vorhersagemodells (VM), erfolgt.

6. Verfahren nach einem der Ansprüche 2 bis 5, wobei das Strukturen-Vorhersagemodell (VM) mit Hilfe eines Trainingsverfahrens gewonnen wird, welches die folgenden Schritte aufweist:
- Erfassen von Trainings-Mess-Projektionsdaten (TRD),
- Durchführen einer iterativen Rekonstruktion (IR) von vorläufigen Trainings-Bilddaten (VTBD) auf Basis der Trainings-Mess-Projektionsdaten (TRD), wobei bei der iterativen Rekonstruktion zusätzlich vorläufige Trainings-Texturmerkmale (VTTM) ermittelt werden,
- Annotieren von Strukturen (AK) in den vorläufigen Trainings-Bilddaten (VTBD),
- Erneutes iteratives Rekonstruieren von vorläufigen Trainings-Bilddaten (VTBD) und Ermitteln von vorläufigen Trainings-Texturmerkmalen (VTTM) unter Berücksichtigung der annotierten Strukturen (AK),
- Einstufen der zuletzt rekonstruierten vorläufigen Trainings-Bilddaten (VTBD) und der zuletzt gewonnenen vorläufigen Trainings-Texturmerkmale (VTTM) als endgültige Trainings-Bilddaten (TBD) und endgültige Trainings-Texturmerkmale (TTM),
- Verwenden der endgültigen Trainings-Bilddaten (TBD) und der endgültigen Trainings-Texturmerkmale (TTM), um das Strukturen-Vorhersagemodell (VM) zu erzeugen.

7. Verfahren nach Anspruch 6, wobei die Schritte des Annotierens von Strukturen (AK) und des erneuten iterativen Rekonstruierens von vorläufigen Trainings-Bilddaten (VTBD) und des erneuten Ermittelns von vorläufigen Trainings-Texturmerkmalen (VTTM) so oft wiederholt werden, bis ein Qualitätskriterium für die ermittelten vorläufigen Trainings-Bilddaten (VTBD) und/oder die ermittelten vorläufigen Trainings-Texturmerkmale (VTTM) erfüllt ist.

8. Verfahren nach einem der Ansprüche 2 bis 7, wobei zur Erzeugung des Strukturen-Vorhersagemodells (VM) auf Basis der endgültigen Trainings-Bilddaten (TBD) und der endgültigen Trainings-Texturmerkmale (TTM) ein maschineller Lernprozess oder ein Deep-Learning-Prozess durchgeführt wird.

9. Verfahren nach einem der Ansprüche 1 bis 8, wobei die Texturmerkmale (TM, VTM, VTTM, TTM) Kantenbilder umfassen.

10. Verfahren nach einem der Ansprüche 1 bis 9 wobei, die Texturmerkmale (TM, VTM, VTTM, TTM) Texturmerkmal-Vektoren aufweisen.

11. Bildrekonstruktionseinrichtung, aufweisend:
- eine Projektionsdatenerfassungseinheit (41) zum Erfassen von Mess-Projektionsdaten (RD, TRD),
- eine Bilddatenrekonstruktionseinheit (42) zum Rekonstruieren von vorläufigen Bilddaten (VBD) und vorläufigen Texturmerkmalen (VTM),
- eine Strukturermittlungseinheit (43) zum Ermitteln von erwarteten vorläufigen Strukturen (VEK), vorzugsweise vorläufigen Konturen, in einem abzubildenden Untersuchungsbereich (FOV) eines Untersuchungsobjekts (O) auf Basis der gewonnenen vorläufigen Texturmerkmale (VTM),
wobei die Bilddatenrekonstruktionseinheit (42) dazu eingerichtet ist, die erwarteten vorläufigen Strukturen (VEK) bei der iterativen Rekonstruktion von vorläufigen Bilddaten (VBD) und vorläufigen Texturmerkmalen (VTM) zu berücksichtigen und die sich aus der iterativen Rekonstruktion zuletzt ergebenden vorläufigen Bilddaten (VBD) und vorläufigen Texturmerkmale (VTM) als die rekonstruierten Bilddaten (BD) und Texturmerkmale (TM) festzulegen.

12. Bildsegmentierungseinrichtung (40), aufweisend:
- eine Bildrekonstruktionseinrichtung nach Anspruch 11,
- eine Segmentierungseinheit (45), welche dazu eingerichtet ist, eine Bildsegmentierung unter Berücksichtigung der ermittelten Texturmerkmale (TM) durchzuführen.

13. Computertomographiesystem (50), aufweisend eine Bildrekonstruktionseinrichtung nach Anspruch 11.

14. Computerprogrammprodukt mit einem Computerprogramm, welches direkt in eine Speichereinheit einer Bildsegmentierungseinrichtung (40) nach Anspruch 12 ladbar ist, mit Programmabschnitten, um alle Schritte des Verfahrens nach einem der Ansprüche 1 bis 10 auszuführen, wenn das Computerprogramm in der Bildsegmentierungseinrichtung (30) ausgeführt wird.

15. Computerlesbares Medium, auf welchem von einer Prozesseinheit einer Bildsegmentierungseinrichtung (40) nach Anspruch 11 einlesbare und ausführbare Programmabschnitte gespeichert sind, um alle Schritte des Verfahrens nach einem der Ansprüche 1 bis 10 auszuführen, wenn die Programmabschnitte von der Prozesseinheit der Bildsegmentierungseinrichtung (40) ausgeführt werden.

## Claims

1. Method for reconstructing image data (BD) and textural features (TM) of an examination region (FOV) to be mapped, having the following steps:
- acquiring scan projection data (RD) from the examination region (FOV) to be mapped,
- carrying out an iterative reconstruction (IR) on the basis of the acquired scan projection data (RD), wherein the following steps occur in the course of iterative reconstruction (IR):
- reconstructing preliminary image data (VBD) and preliminary textural features (VTM),
- determining anticipated preliminary structures, preferably anticipated preliminary contours (VEK), in the examination region (FOV) on the basis of the obtained preliminary textural features (VTM),
- taking into account the anticipated preliminary structures during iterative reconstruction of preliminary image data (VBD) and preliminary textural features (VTM).
- defining the preliminary image data (VBD) and preliminary textural features (VTM) most recently resulting from the iterative reconstruction as the reconstructed image data (BD) and textural features (TM).

2. Method according to claim 1, wherein determining the anticipated preliminary structures (VEK) in the examination region (FOV) also takes place on the basis of the reconstructed preliminary image data (VBD) and/or a structure prediction model (VM).

3. Method according to claim 1 or 2, wherein the steps are repeated in the course of iterative reconstruction (IR) until a quality criterion is met for the determined preliminary image data (VBD) and preliminary textural features (VTM).

4. Method for segmentation of an examination region (FOV) to be mapped, having the steps:
- reconstructing image data (BD) and textural features (TM) using the method according to one of claims 1 to 3,
- segmentation of the examination region (FOV) to be mapped while taking into account the textural features (TM) determined.

5. Method according to claim 4, wherein segmentation of the examination region (FOV) to be mapped also takes place while taking into account the image data (BD) determined and/or a structure prediction model, preferably a contour prediction model (VM).

6. Method according to one of claims 2 to 5, wherein the structure prediction model (VM) is obtained with the aid of a training method which has the following steps:
- acquiring training scan projection data (TRD),
- carrying out an iterative reconstruction (IR) of preliminary training image data (VTBD) on the basis of the training scan projection data (TRD), wherein preliminary training textural features (VTTM) are also determined during iterative reconstruction,
- annotating structures (AK) in the preliminary training image data (VTBD),
- renewed iterative reconstructing of preliminary training image data (VTBD) and determining preliminary training textural features (VTTM) while taking into account the annotated structures (AK),
- classifying the most recently reconstructed preliminary training image data (VTBD) and the most recently obtained preliminary training textural features (VTTM) as the definitive training image data (TBD) and definitive training textural features (TTM),
- using the definitive training image data (TBD) and the definitive training textural features (TTM) to generate the structure prediction model (VM).

7. Method according to claim 6, wherein the steps of annotating structures (AK) and renewed iterative reconstruction of preliminary training image data (VTBD) and renewed determining of preliminary training textural features (VTTM) are repeated until a quality criterion is met for the determined preliminary training image data (VTBD) and/or the determined preliminary training textural features (VTTM).

8. Method according to one of claims 2 to 7, wherein a machine learning process or a deep-learning process is carried out to generate the structure prediction model (VM) on the basis of the definitive training image data (TBD) and the definitive training textural features (TTM).

9. Method according to one of claims 1 to 8, wherein the textural features (TM, VTM, VTTM, TTM) comprise edge images.

10. Method according to one of claims 1 to 9, wherein the textural features (TM, VTM, VTTM, TTM) have textural feature vectors.

11. Image reconstruction device, having:
- a projection data acquisition unit (41) for acquiring scan projection data (RD, TRD),
- an image data reconstruction unit (42) for reconstructing preliminary image data (VBD) and preliminary textural features (VTM),
- a structure determining unit (43) for determining anticipated preliminary structures (VEK), preferably preliminary contours, in an examination region (FOV) of an examination object (O) to be mapped on the basis of the obtained preliminary textural features (VTM),
wherein the image data reconstruction unit (42) is adapted to take into account the anticipated preliminary structures (VEK) during iterative reconstruction of preliminary image data (VBD) and preliminary textural features (VTM) and to define the preliminary image data (VBD) and preliminary textural features (VTM) most recently resulting from the iterative reconstruction as the reconstructed image data (BD) and textural features (TM).

12. Image segmentation device (40), having:
- an image reconstruction device according to claim 11,
- a segmentation unit (45) which is adapted to carry out image segmentation while taking into account the textural features (TM) determined.

13. Computer tomography system (50), having an image reconstruction device according to claim 11.

14. Computer program product comprising a computer program which can be loaded directly into a storage unit of an image segmentation device (40) according to claim 12, having program segments to carry out all steps of the method according to one of claims 1 to 10 when the computer program is run in the image segmentation device (30).

15. Computer-readable medium on which program segments, which can be read and executed by a processor unit of an image segmentation device (40) according to claim 11, are stored to carry out all steps of the method according to one of claims 1 to 10 when the program segments are executed by the processor unit of the image segmentation device (40).

## Revendications

1. Procédé de reconstruction de données (BD) d'image et de caractéristiques (TM) de texture d'une région (FOV) d'étude à représenter, qui a les stades dans lesquels :
- on détecte des données (RD) de projection de mesure de la région (FOV) d'étude à représenter,
- on effectue une reconstruction (IR) itérative sur la base des données (RD) de projection de mesure qui ont été détectées, dans lequel, dans le cadre de la reconstruction (IR) itérative, on effectue les stades suivants :
- reconstruction de données (VBD) d'image provisoires et de caractéristiques (VTM) de texture provisoires,
- détermination de structures provisoires escomptées, de préférence de contours (VEK) provisoires escomptés, dans la région (FOV) d'étude sur la base des caractéristiques (VTM) de texture provisoires qui ont été obtenues,
- prise en compte des structures provisoires escomptées dans la reconstruction itérative de données (VBD) d'image provisoires et de caractéristiques (VTM) de texture provisoires,
- on établit les données (VBD) d'image provisoires et les caractéristiques (VTM) de texture provisoires ressortant en dernier de la reconstruction itérative comme données (BD) d'image et caractéristiques (TM) de texture reconstruites.

2. Procédé suivant la revendication 1, dans lequel la détermination des structures (VEK) escomptées dans la région (FOV) d'étude s'effectue supplémentairement sur la base des données (VBD) d'image provisoires reconstruites et/ou d'un modèle (VM) de prédiction de structure.

3. Procédé suivant la revendication 1 ou 2, dans lequel on répète les stades, dans le cadre de la reconstruction (IR) itérative, jusqu'à ce qu'un critère de qualité des données (VBD) d'image provisoires et des caractéristiques (VTM) de structure provisoires qui ont été déterminées soit satisfait.

4. Procédé de segmentation d'une région (FOV) d'étude à représenter, comprenant les stades dans lesquels :
- on reconstruit des données (BD) d'image et des caractéristiques (TM) de texture en utilisant le procédé suivant l'une des revendications 1 à 3,
- on segmente la région (FOV) d'étude à représenter en tenant compte des caractéristiques (TM) de textures qui ont été déterminées.

5. Procédé suivant la revendication 4, dans lequel la segmentation de la région (FOV) d'étude à représenter s'effectue supplémentairement en tenant compte des données (BD) d'image déterminées et/ou d'un modèle de prédiction de structure, de préférence d'un modèle (VM) de prédiction de contour.

6. Procédé suivant l'une des revendications 2 à 5, dans lequel on obtient le modèle (VM) de prédiction de structure à l'aide d'un procédé d'apprentissage, qui a les stades de procédé suivants :
- on détecte des données (TRD) de projection de mesure d'apprentissage,
- on effectue une reconstruction (IR) itérative de données (VTBD) d'image d'apprentissage provisoires sur la base des données (TRD) de projection de mesure d'apprentissage, dans lequel, dans la reconstruction itérative, on détermine supplémentairement des caractéristiques (VTTM) de texture d'apprentissage provisoires,
- on annote des structures (AK) dans les données (VTBD) d'image d'apprentissage provisoires,
- on reconstruit itérativement à nouveau des données (VTBD) d'image d'apprentissage provisoires et on détermine des caractéristiques (VTTM) de texture d'apprentissage provisoires en tenant compte des structures (AK) annotées,
- on classe les données (VTBD) d'image d'apprentissage provisoires reconstruites en dernier et les caractéristiques (VTTM) d'apprentissage provisoires obtenues en dernier comme données (TDD) d'image d'apprentissage définitives et caractéristiques (TTM) de texture d'apprentissage définitives,
- on utilise les données (TBD) d'image d'apprentissage définitives et les caractéristiques (TTM) de texture d'apprentissage définitives, pour produire le modèle (VM) de prédiction de structure.

7. Procédé suivant la revendication 6, dans lequel on répète les stades de l'annotation de structure (AK) et de la reconstruction itérative renouvelée de données (VTBD) d'image d'apprentissage provisoires et de la détermination renouvelée de caractéristiques (VTTM) de texture d'apprentissage provisoires, jusqu'à ce qu'un critère de qualité des données (VTBD) d'image d'apprentissage provisoires déterminées et/ou des caractéristiques (VTTM) de texture d'apprentissage provisoires déterminées soit satisfait.

8. Procédé suivant l'une des revendications 2 à 7, dans lequel, pour produire le module (VM) provisoire de structure sur la base des données (TBD) d'image d'apprentissage définitives et des caractéristiques (TTM) de texture d'apprentissage définitives, on effectue une opération d'apprentissage automatique ou une opération deep-learning.

9. Procédé suivant l'une des revendications 1 à 8, dans lequel les caractéristiques (TM, VTM, VTTM, TTM) de texture comprennent des images de bord.

10. Procédé suivant l'une des revendications 1 à 9, dans lequel les caractéristiques (TM, VTM, VTTM, TTM) de texture ont des vecteurs de caractéristiques de texture.

11. Dispositif de reconstruction d'image, comportant :
- une unité (41) de données de projection pour détecter des données (RD, TRD) de projection de mesure,
- une unité (42) de reconstruction de données d'image pour reconstruire des données (VBD) d'image provisoires et des caractéristiques (VTM) de texture provisoires,
- une unité (43) de détermination de structure pour déterminer des structures (VEK) provisoires escomptées, de préférence des contours provisoires, dans une région (FOV) d'étude à représenter d'un objet (O) d'étude sur la base des caractéristiques (VTM) de texture provisoires qui ont été obtenues,
l'unité (42) de reconstruction de données d'image étant conçue pour tenir compte des structures (VEK) provisoires escomptées dans la reconstruction itérative de données (VDB) d'image provisoires et de caractéristiques (VTM) de texture provisoires et pour établir les données (VBD) d'image provisoires et les caractéristiques (VTM) de texture provisoires obtenues en dernier dans la reconstruction itérative comme données (BD) d'image et caractéristiques (TM) de texture reconstruites.

12. Dispositif (40) de segmentation d'image, comportant :
- un dispositif de reconstruction d'image suivant la revendication 11,
- une unité (45) de segmentation conçue pour effectuer une segmentation d'image en tenant compte des caractéristiques (TM) de textures qui ont été déterminées.

13. Système (50) de tomographie par ordinateur, comportant un dispositif de reconstruction d'image suivant la revendication 11.

14. Produit de programme d'ordinateur ayant un programme d'ordinateur, qui peut être chargé directement dans une unité de mémoire d'un dispositif (40) de segmentation d'image suivant la revendication 12, ayant des parties de programme pour exécuter tous les stades du procédé suivant l'une des revendications 1 à 1, lorsque le programme d'ordinateur est réalisé dans le dispositif (30) de segmentation d'image.

15. Support déchiffrable par ordinateur, sur lequel sont mémorisées des parties de programme pouvant être déchiffrées et réalisées par une unité de traitement d'un dispositif (40) de segmentation d'image suivant la revendication 11, pour effectuer tous les stades du procédé suivant l'une des revendications 1 à 10, lorsque les parties de programme sont réalisées par l'unité de traitement du dispositif (40) de segmentation d'image.
